# EUROPEAN PATENT APPLICATION

(11) **EP 4 736 904 A2**
(43) Date of publication of application: **06.05.2026**
(21) Application number: 25206016.5
(22) Date of filing: 01.10.2025
(51) Int. Cl.: A61M 1/16

(54) **BLOOD OXYGENATOR**

(30) Priority: 14.10.2024 US 202418914703
(71) Applicant: Sorin Group Italia S.r.l., 20159 Milano (IT)
(72) Inventor: PANDOLFINI, Clara, I-46048 Roverbella (MN) (IT); MADONIA, Francesco, I-90135 Palermo (PA) (IT); GALAVOTTI, Andrea, I-41037 Mirandola (MO) (IT)
(74) Representative: Buzzi, Notaro & Antonielli d'Oulx S.p.A.

(57) **Abstract**

An oxygenator including a housing defining an interior having improved gas exchange fluid pathways and/or heat exchange fluid pathways is disclosed. In some instances, the oxygenator includes a gas exchange fluid pathway for passing a gas sequentially from a gas exchange fluid inlet, through one or more layers of gas exchange fibers extending in a first direction, through one or more layers of gas exchange fibers extending in a second direction, and out a gas exchange fluid outlet. In some instances, the oxygenator includes a partition dividing the interior between a gas exchange chamber and a gas-and-heat exchange chamber. In some instances, the housing includes an insulating fluid barrier chamber configured to be filled with a flowing heat exchange fluid from the heat exchange fluid inlet to provide an insulating barrier between outflow ends of the gas exchange fibers and the gas exchange fluid outlet.

## Description

### TECHNICAL FIELD

The present disclosure pertains to extracorporeal blood conditioning devices, such as blood oxygenators. More particularly, the present disclosure pertains to blood oxygenators, the construction and use thereof, which provide improved fluid flow paths therethrough.

### BACKGROUND

Blood perfusion typically entails pumping blood through the blood vessels of the body of a patient using one or more pumps in an extracorporeal circuit that is interconnected with the vascular system of the patient. The extracorporeal circuit typically includes an oxygenator, such as a hollow fiber blood oxygenator, used to exchange oxygen (O₂) and carbon dioxide (CO₂) in extracorporeal circulation of the blood from the patient. Cardiopulmonary bypass surgery typically requires a perfusion system that provides for the temporary cessation of the heart to create a still operating field by replacing the function of the heart and lungs. Such isolation allows for the surgical correction of vascular stenosis, valvular disorders, congenital heart defects, and other medical procedures. In perfusion systems used for cardiopulmonary bypass surgery, an extracorporeal blood circuit is established that includes at least one pump and an oxygenation device to replace the functions of the heart and lungs.

More specifically, in cardiopulmonary bypass procedures oxygen-poor blood, i.e., venous blood, is gravity-drained or vacuum suctioned from a large vein entering the heart or other veins in the body (e.g., femoral) and is transferred through a venous line in the extracorporeal circuit. The venous blood is pumped to an oxygenator that provides for oxygen transfer to the blood. Oxygen may be introduced into the blood by transfer across a membrane or, less frequently, by bubbling oxygen through the blood. Concurrently, carbon dioxide is removed across the membrane. The oxygenated blood is filtered and then returned through an arterial line to the aorta, femoral artery, or other artery of the patient.

There are many drawbacks to currently available oxygenators. For example, there is an ongoing need to improve the efficiency of oxygen exchange from the gas-exchange fibers into the blood passing through the oxygenator. Additionally, during the use of an oxygenator, vapor condensation may occur within the gas-exchange fibers of the oxygenator, leading to a progressive decrease in performance of gas exchange within the oxygenator. Accordingly, there is an ongoing need for alternative oxygenator configurations which may increase the efficiency of the oxygenator, reduce vapor condensation, or otherwise improve the performance of the oxygenator.

### BRIEF SUMMARY

This disclosure provides design, material, manufacturing method, and use alternatives for blood oxygenators.

A first example is an oxygenator including a housing defining an internal chamber. A plurality gas exchange fibers are disposed within in the internal chamber. The plurality of gas exchange fibers include one or more layers of gas exchange fibers extending in a first direction and one or more layers of gas exchange fibers extending in a second direction. The second direction is generally orthogonal to the first direction. The oxygenator also includes a gas exchange fluid inlet and a gas exchange fluid outlet. A gas exchange fluid pathway is provided for passing a gas through the plurality of gas exchange fibers to oxygenate blood within the internal chamber. The gas exchange fluid pathway passes sequentially from the gas exchange fluid inlet, through the one or more layers of gas exchange fibers extending in the first direction, through the one or more layers of gas exchange fibers extending in the second direction, and out the gas exchange fluid outlet.

Alternatively or additionally to any of the examples herein, in another example, the housing includes a first side, a second side opposite the first side, a third side extending between the first side and the second side, and a fourth side extending between the first side and the second side, and opposite the third side. The gas exchange fluid pathway passes through the one or more layers of gas exchange fibers extending in the first direction from the fourth side to the third side, and then the gas exchange fluid pathway passes through the one or more layers of gas exchange fibers extending in the second direction from the first side to the second side.

Alternatively or additionally to any of the examples herein, a first velocity of a gas exchange fluid flowing through the one or more layers of gas exchange fibers extending in the first direction from the fourth side to the third side is greater than a second velocity of the gas exchange fluid flowing through the one or more layers of gas exchange fibers extending in the second direction from the first side to the second side.

Alternatively or additionally to any of the examples herein, the first velocity is 1.25 times or more the velocity of the second velocity.

Alternatively or additionally to any of the examples herein, in another example, the oxygenator includes a gas exchange fluid passage defined in the housing between gas outflow ends of the one or more layers of gas exchange fibers extending in the first direction and gas inflow ends of the one or more layers of gas exchange fibers extending in the second direction.

Alternatively or additionally to any of the examples herein, in another example, the internal chamber is a gas exchange chamber, and the housing includes a second internal chamber which is a gas-and-heat exchange chamber, wherein the gas-and-heat exchange chamber includes a plurality of gas exchange fibers disposed within the gas-and-heat exchange chamber and a plurality of heat exchange fibers disposed within the gas-and-heat exchange chamber.

Alternatively or additionally to any of the examples herein, in another example, the oxygenator includes a partition between the gas exchange chamber and the gas-and-heat exchange chamber.

Alternatively or additionally to any of the examples herein, in another example, the housing includes a blood inlet in fluid communication with the gas-and-heat exchange chamber and a blood outlet in fluid communication with the gas exchange chamber.

Alternatively or additionally to any of the examples herein, in another example, a blood flow path passes sequentially from the blood inlet, through the gas-and-heat exchange chamber, through the gas exchange chamber, and out the blood outlet.

Alternatively or additionally to any of the examples herein, in another example, the plurality of heat exchange fibers in the gas-and-heat exchange chamber are arranged in the first direction, and the plurality of gas exchange fibers in the gas-and-heat exchange chamber are arranged in the second direction.

Alternatively or additionally to any of the examples herein, in another example, the housing includes a first side, a second side opposite the first side, a third side extending between the first side and the second side, and a fourth side extending between the first side and the second side, and opposite the third side. The gas exchange fluid pathway passes through the one or more layers of gas exchange fibers extending in the first direction from the fourth side to the third side, and then the gas exchange fluid pathway passes through the one or more layers of gas exchange fibers extending in the second direction from the first side to the second side.

Alternatively or additionally to any of the examples herein, in another example, the second side includes an insulating fluid barrier chamber configured to be filled with a flowing heat exchange fluid.

Alternatively or additionally to any of the examples herein, in another example, the housing includes a heat exchange fluid port in fluid communication with the insulating fluid barrier chamber.

Alternatively or additionally to any of the examples herein, in another example, the heat exchange fluid port and the gas exchange fluid outlet are both located on the second side of the housing.

Alternatively or additionally to any of the examples herein, in another example, the insulating fluid barrier chamber is positioned between an outflow of the one or more layers of gas exchange fibers extending in the second direction at the second side and an exterior of the second side of the housing.

Another example is an oxygenator including a housing defining an interior. The interior includes a gas exchange chamber and a gas-and-heat exchange chamber. A partition is located between the gas exchange chamber and the gas-and-heat exchange chamber. A plurality gas exchange fibers are disposed within the gas exchange chamber. A plurality of gas exchange fibers are disposed within the gas-and-heat exchange chamber. A plurality of heat exchange fibers are disposed within the gas-and-heat exchange chamber.

Alternatively or additionally to any of the examples herein, in another example, the plurality of gas exchange fibers disposed within the gas exchange chamber include one or more layers of gas exchange fibers extending in a first direction and one or more layers of gas exchange fibers extending in a second direction. The second direction may be generally orthogonal to the first direction.

Alternatively or additionally to any of the examples herein, in another example, a gas exchange fluid pathway for passing a gas through the plurality of gas exchange fibers in the gas exchange chamber to oxygenate blood within the in the gas exchange chamber sequentially passes through the one or more layers of gas exchange fibers extending in the first direction and thereafter through the one or more layers of gas exchange fibers extending in the second direction.

Alternatively or additionally to any of the examples herein, in another example, a first velocity of a gas exchange fluid flowing through the one or more layers of gas exchange fibers extending in the first direction is greater than a second velocity of the gas exchange fluid flowing through the one or more layers of gas exchange fibers extending in the second direction.

Alternatively or additionally to any of the examples herein, in another example, the oxygenator includes a gas exchange fluid passage defined in the housing between gas outflow ends of the one or more layers of gas exchange fibers extending in the first direction and gas inflow ends of the one or more layers of gas exchange fibers extending in the second direction.

Alternatively or additionally to any of the examples herein, in another example, the plurality of heat exchange fibers arranged in the gas-and-heat exchange chamber include one or more layers of heat exchange fibers extending in the first direction, and the plurality of gas exchange fibers arranged in the gas-and-heat exchange chamber include one or more layers of gas exchange fibers extending in the second direction. The second direction may be generally orthogonal to the first direction.

Another example is an oxygenator including a housing having a first side, a second side opposite the first side, a third side extending between the first side and the second side, and a fourth side extending between the first side and the second side, and opposite the third side. The housing defines an interior. A plurality of gas exchange fibers are disposed within the interior of the housing. The plurality of gas exchange fibers extend between the first side and the second side with gas outflow from the plurality of gas exchange fibers along the second side. A plurality of heat exchange fibers are disposed within the interior of the housing. The oxygenator includes a gas exchange fluid inlet in fluid communication with the plurality of gas exchange fibers and a gas exchange fluid outlet in fluid communication with the plurality of gas exchange fibers. The oxygenator also includes a heat exchange fluid inlet in fluid communication with the heat exchange fibers and a heat exchange fluid outlet in fluid communication with the heat exchange fibers. The second side of the housing includes an insulating fluid barrier chamber configured to be filled with a flowing heat exchange fluid from the heat exchange fluid inlet.

Alternatively or additionally to any of the examples herein, in another example, the insulating fluid barrier is fluidly arranged between the heat exchange fluid inlet and inflow ends of the plurality of heat exchange fibers.

Alternatively or additionally to any of the examples herein, in another example, the inflow ends of the plurality of heat exchange fibers are located along the fourth side, and wherein the plurality of heat exchange fibers extend between the fourth side and the third side.

Another example is an oxygenator including a housing defining an interior. A plurality gas exchange fibers are disposed within in the interior. The plurality of gas exchange fibers include one or more layers of gas exchange fibers extending in a first direction and one or more layers of gas exchange fibers extending in a second direction. The second direction is generally orthogonal to the first direction. The oxygenator also includes a gas exchange fluid inlet and a gas exchange fluid outlet. A gas exchange fluid pathway is provided for passing a gas through the plurality of gas exchange fibers to oxygenate blood within the interior of the housing. The gas exchange fluid pathway passes from the gas exchange fluid inlet, through the plurality of gas exchange fibers, and out the gas exchange fluid outlet. A first velocity of a gas exchange fluid flowing through the one or more layers of gas exchange fibers extending in the first direction is greater than a second velocity of the gas exchange fluid flowing through the one or more layers of gas exchange fibers extending in the second direction.

Alternatively or additionally to any of the examples herein, in another example, the first velocity is 1.25 times or more the velocity of the second velocity.

The above summary of some embodiments is not intended to describe each disclosed embodiment or every implementation of the present disclosure. The Figures, and Detailed Description, which follow, more particularly exemplify some of these embodiments.

### BRIEF DESCRIPTION OF THE DRAWINGS

The disclosure may be more completely understood in consideration of the following detailed description in connection with the accompanying drawings, in which:
FIG. 1 is a perspective top view of an example blood oxygenator;
FIG. 2 is a perspective bottom view of the blood oxygenator of FIG. 1;
FIG. 3 is an exploded view of the blood oxygenator of FIG. 1;
FIG. 4 is a perspective view taken through a cross-section of the blood oxygenator taken from a first side to a second side of the blood oxygenator;
FIG. 5 is a perspective view taken through a cross-section of the blood oxygenator from a third side to a fourth side of the blood oxygenator;
FIG. 6 is a perspective top view of the blood oxygenator with the top panel removed to view an interior of the blood oxygenator;
FIG. 7 is a perspective top view of the blood oxygenator with the top panel and side panel removed to view the internal chambers of the blood oxygenator;
FIG. 8 is a cross-sectional view through a first, gas-and-heat exchange chamber of the blood oxygenator illustrating a gas exchange fluid flow path and a heat exchange fluid flow path therethrough;
FIG. 9 is a perspective view of a fiber mat including heat exchange fluid fiber layers extending in a first direction arranged with gas exchange fiber layers extending in a second direction for use in the gas-and-heat exchange chamber;
FIG. 10 is a perspective bottom view of the blood oxygenator with the bottom panel removed to view an internal chamber;
FIG. 11 is a perspective bottom view of the blood oxygenator with the bottom panel and side panel removed to view the internal chambers of the blood oxygenator;
FIG. 12 is a cross-sectional view through a second, gas exchange chamber of the blood oxygenator illustrating a gas exchange fluid flow path therethrough; and
FIG. 13 is a perspective view of a fiber mat including gas exchange fluid fiber layers extending in a first direction arranged with gas exchange fiber layers extending in a second direction for use in the gas exchange chamber.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The terms "top," "up," "upper," and "upward," and variations thereof, are used throughout this disclosure for the sole purpose of clarity of description and are only intended to refer to a relative direction (i.e., a certain direction that is to be distinguished from another direction), and are not meant to be interpreted to mean an absolute direction. Similarly, the terms "bottom," "down," "lower," and "downward," and variations thereof, are used throughout this disclosure for the sole purpose of clarity of description and are only intended to refer to a relative direction that is at least approximately opposite a direction referred to by one or more of the terms "top," "up," "upper," and "upward," and variations thereof.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The terms "monolithic" and "unitary" shall generally refer to an element or elements made from or consisting of a single structure or base unit/element. A monolithic and/or unitary element shall exclude structure and/or features made by assembling or otherwise joining multiple discrete structures or elements together.

The term "sequentially", as used herein, is intended to refer to an ordering of elements, paths, steps, events, etc. in the order listed, but does not exclude additional elements, paths, steps, events, etc. before, between, or after any of the listed elements, steps, events, etc. unless expressly indicated that the expressly listed elements, paths, steps, events, etc. are directly sequentially arranged.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For example, a reference to some features may be equally referred to all instances and quantities beyond one of said feature(s) unless clearly stated to the contrary. As such, it will be understood that the following discussion may apply equally to any and/or all of the components for which there are more than one within the device, etc. unless explicitly stated to the contrary.

The following detailed description should be read with reference to the drawings in which similar structures in different drawings are numbered the same. The drawings, which are not necessarily to scale, depict illustrative embodiments and are not intended to limit the scope of the disclosure.

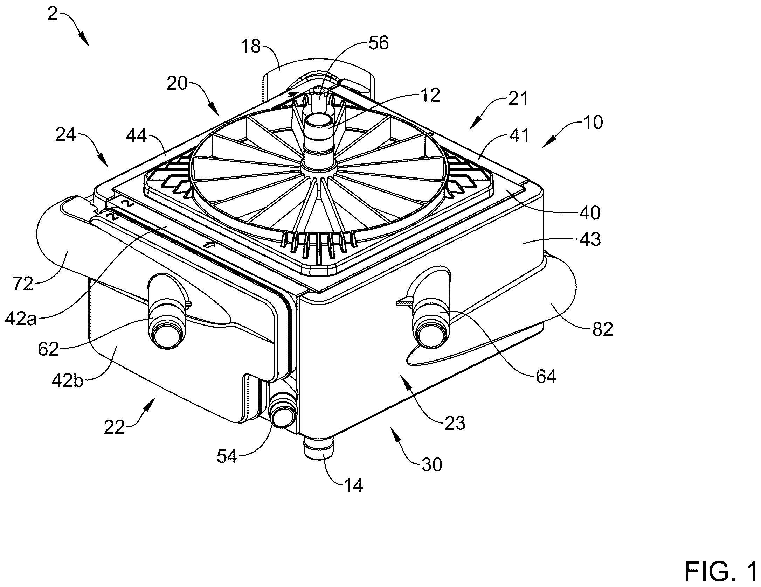

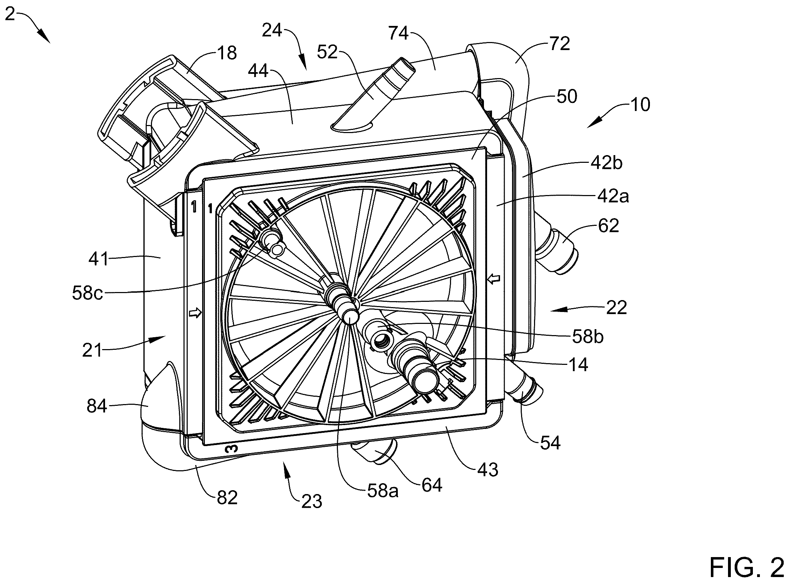

FIG. 1 is a perspective top view of an oxygenator 2 for conditioning blood in extracorporeal circulation, such as with an extracorporeal life support (ECLS) system, for example, while FIG. 2 is a perspective bottom view of the oxygenator 2. The oxygenator 2 may include a housing 10 having a top 20 and a bottom 30, opposite the top 20. The oxygenator 2 may include a plurality of sides extending around a perimeter of the housing 10 between the top 20 and the bottom 30. For example, the housing 10 may include a first side 21, a second side 22 opposite the first side 21, a third side 23, and a fourth side 24 opposite the third side 23. The third side 23 and the fourth side 24 may extend between the first side 21 and the second side 22. Thus, the first side 21 may be adjacent the third side 23 and the fourth side 24, the second side 22 may be adjacent the third side 23 and the fourth side 24, the third side 23 may be adjacent the first side 21 and the second side 22, and the fourth side 24 may be adjacent the first side 21 and the second side 22. The housing 10 may also include a connector 18, configured to connect the oxygenator 2 to a component of an ECLS, a blood perfusion system, or other medical system. For example, the connector 18 may be used to connect the oxygenator 2, such as via a dedicated bracket, to a mast or pole of a blood perfusion system.

The housing 10 may be a rigid housing and/or the housing 10 may be constructed from a substantially rigid material. In some instances, the housing 10 may be formed of a plurality of panels, e.g.., polymeric panels, assembled together to enclose an interior of the housing 10. For example, the housing 10 may include a top panel 40 arranged on the top 20 of the housing 10 and a bottom panel 50 arranged on the bottom 30 of the housing 10. The top panel 40 may define an upper exterior surface of the housing 10 and/or the bottom panel 50 may define a lower exterior surface of the housing 10.

Additionally, the housing 10 may include a first side panel 41 defining, arranged on, or otherwise extending along the first side 21 of the housing 10, a second side panel 42 (collectively the second side inner panel 42a and the second side outer panel 42b, as described further herein) defining, arranged on, or otherwise extending along the second side 22 of the housing 10, a third side panel 43 defining, arranged on, or otherwise extending along the third side 23 of the housing 10, and/or a fourth side panel 44 defining, arranged on, or otherwise extending along the fourth side 24 of the housing 10. In some instances, each of the panels may be formed separately and then assembled to form the housing 10. In other instances, one or more of the panels may be formed together with one or more of the panels, and thereafter assembled with one or more additional panels to form the housing 10. Other arrangements and configurations for the construction of the housing 10 are also contemplated.

The housing 10 may include a blood inlet 12 in fluid communication with an interior of the housing 10 to permit the flow of venous, deoxygenated blood into the oxygenator 2 from a patient, and the housing 10 may include a blood outlet 14 in fluid communication with the interior of the housing 10 to permit the flow of arterial, oxygenated blood from the oxygenator to a patient. Blood inflow tubing (not shown) may be connected to the blood inlet 12 and/or blood outflow tubing (not shown) may be connected to the blood outlet 14 during use of the oxygenator 2. In some instances, the blood inlet 12 may extend from the top 20 of the housing 10, such as extend from the top panel 40 of the housing 10. In some instances, the blood outlet 14 may extend from the bottom 30 of the housing 10, such as extend from the bottom panel 50 of the housing 10. Other arrangements and configurations of the blood inlet 12 and blood outlet 14 are also contemplated.

The housing 10 may include a gas inlet 52 in fluid communication with a gas exchange structure (e.g., a gas exchange fiber mat or section) within the interior of the housing 10, and the housing 10 may include a gas outlet 54 in fluid communication with the gas exchange structure within the interior of the housing 10. For example, the gas inlet 52 may be in fluid communication with the lumens of a plurality of gas exchange fibers to provide a gas (e.g., oxygen) to oxygenate blood passing through the oxygenator 2, while the gas outlet 54 may be in fluid communication with the lumens of the plurality of gas exchange fibers to remove gas (e.g., carbon dioxide) taken from the blood. In some instances, the gas inlet 52 may extend from the fourth side 24 of the housing 10, such as extend from the fourth side panel 44 of the housing 10. In some instances, the gas outlet 54 may extend from the second side 22, such as from the second side inner panel 42a of the housing 10. Thus, in some instances, the gas inlet 52 and the gas outlet 54 may extend from adjacent sides of the housing 10. Other arrangements and configurations of the blood inlet 12 and blood outlet 14 are also contemplated.

The housing 10 may include a first heat exchange fluid port 62, which in some instances may be referred to as a heat exchange fluid inlet, in fluid communication with a heat exchange structure (e.g., a heat exchange fiber mat or section) within the interior of the housing 10, and the housing 10 may include a second heat exchange fluid port 64, which in some instances may be referred to as a heat exchange fluid outlet, in fluid communication with the heat exchange structure within the interior of the housing 10. For example, the heat exchange fluid port 62, when acting as a heat exchange fluid inlet, may be in fluid communication with the lumens of a plurality of heat exchange fibers to provide a heat exchange fluid (e.g., water, or other liquid) to heat/cool the blood passing through the oxygenator 2, while the heat exchange fluid port 64, when acting as a heat exchange fluid outlet, may be in fluid communication with the lumens of the plurality of heat exchange fibers to remove the heat exchange fluid (e.g., water, or other liquid) from the oxygenator 2. In other instances, the direction of flow of the heat exchange fluid through the oxygenator 2 (e.g., through the plurality of heat exchange fibers) may be reversed, such that the second heat exchange fluid port 64 acts as a heat exchange fluid inlet and the first heat exchange fluid port 62 acts as a heat exchange fluid outlet. In some instances, the first heat exchange fluid port 62 (e.g., the heat exchange fluid inlet) may extend from the second side 22 of the housing 10, such as extend from the second side outer panel 42b of the housing 10. In some instances, the second heat exchange fluid port 64 (e.g., the heat exchange fluid outlet) may extend from the third side 23, such as from the third side panel 43 of the housing 10. Thus, in some instances, the first heat exchange fluid port 62 and the second heat exchange fluid port 64 may extend from adjacent sides of the housing 10. Other arrangements and configurations of the heat exchange fluid ports 62, 64 are also contemplated.

The housing 10 may include one or more additional, auxiliary ports, in some instances. For example, the housing 10 may include an auxiliary port 56 extending from the top panel 40, a first auxiliary port 58a extending from the bottom panel 50, a second auxiliary port 58b extending from the bottom panel 50, and/or a third auxiliary portion 58c extending from the bottom panel 50. In some instances, the first auxiliary port 58a may be referred to as a cardioplegia port, configured to supply blood back into the heart cavity of a patient. In some instances, the second auxiliary port 58b may be referred to as a temperature control port, couplable to a temperature probe to measure the temperature of the blood passing through the oxygenator 2. In some instances, the third auxiliary port 58c may be referred to as a purge port outlet. In some instances, the auxiliary port 56 may be referred to as a purge port inlet. The purge port inlet and the purge port outlet may be configured to collectively purge air bubbles from within the oxygenator 2. More or fewer ports, their use, and the arrangement and configuration thereof, are also contemplated.

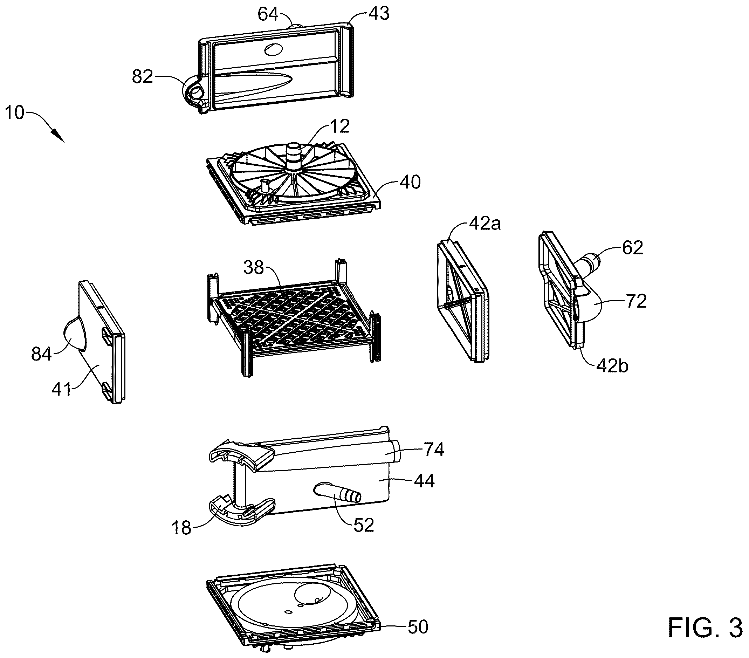

FIG. 3 is an exploded view of the housing 10 of the oxygenator 2, in which the top panel 40, the bottom panel 50, the first side panel 41, the second side inner panel 42a, the second side outer panel 42b, the third side panel 43, and the fourth side panel 44 are separated from one another. As shown in FIG. 3, the interior of the housing 10 may include a divider or partition 38, dividing the interior of the housing 10 into multiple chambers or compartments. The partition 38 may extend across the interior of the housing 10 between the first and second sides 21, 22, and the partition may extend across the interior of the housing between the third and fourth sides 23, 24, dividing the interior of the housing 10 into an upper chamber or compartment defined between the top panel 40 and the upper surface of the partition 38, and a lower chamber or compartment defined between the bottom panel 50 and the lower surface of the partition 38. The partition 38 may be fenestrated such that the partition 38 includes a plurality of apertures or openings extending through the partition 38 from the upper surface of the partition 38 to the lower surface of the partition 38 to permit blood to pass through the partition 38 from the upper chamber to the lower chamber, as will be described further herein.

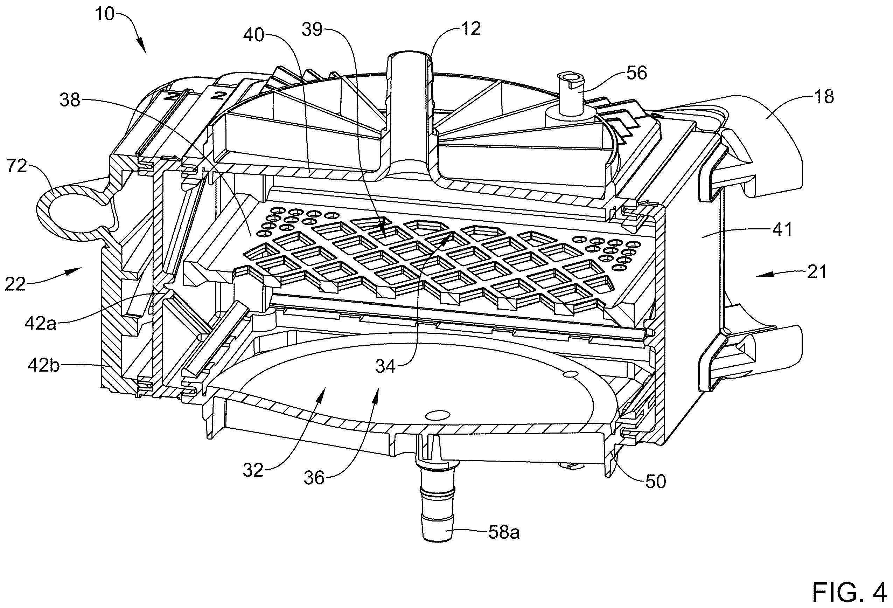

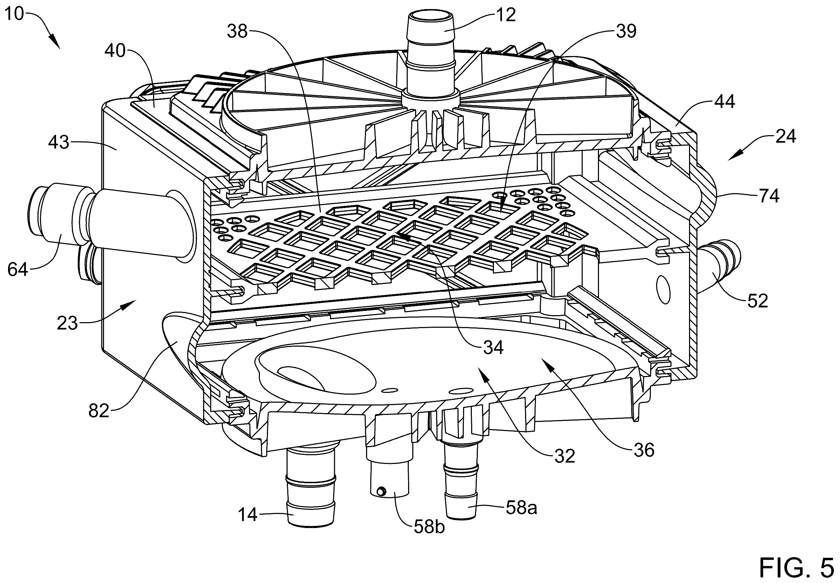

FIG. 4 is a perspective view taken through a cross-section of the oxygenator 2 taken from the first side 21 to the second side 22 of the oxygenator 2, wherein FIG. 5 is a perspective view taken through a cross-section of the oxygenator 2 from the third side 23 to the fourth side of the oxygenator 2. The cross-section of FIG. 4 is taken perpendicular to the cross-section of FIG. 5. As shown in FIGS. 4 and 5, the partition 38 may divide the interior 32 of the housing 10 into a first, or upper chamber 34 and a second, or lower chamber 36. As can be seen in FIGS. 4 and 5, the first chamber 34 may be defined between the top panel 40 and the upper surface of the partition 38, whereas the second chamber 36 may be defined between the bottom panel 50 and the lower surface of the partition 38. As shown in FIG. 4, the partition 38 may include first and second opposing lateral edges that extend to, join with, interlock with, or otherwise engage the first and second side panels 41, 42 respectively. As shown in FIG. 5, the partition 38 may include third and fourth opposing lateral edges that extend to, join with, interlock with, or otherwise engage the third and fourth side panels 43, 44 respectively. As noted above, the partition 38 may be fenestrated such that the partition 38 includes a plurality of apertures or openings 39 extending through the partition 38 from the upper surface of the partition 38 to the lower surface of the partition 38 to permit blood to pass through the partition 38 from the first chamber 34 to the second chamber 36.

The blood inlet 12 may be in fluid communication (e.g., direct fluid communication) with the first chamber 34 to direct blood into the first chamber 34. The blood outlet 14 may be in fluid communication (e.g., direct fluid communication) with the second chamber 36 to collect blood exiting from the second chamber 36. Accordingly, blood may flow sequentially from the blood inlet 12 into the first chamber 34, then pass through the plurality of apertures or openings 39 of the partition 38 into the second chamber 36, and then exit the second chamber 36 through the blood outlet 14. In other words, a blood flow pathway may be defined sequentially from the blood inlet 12, through the first chamber 34, through or across the partition 38 (e.g., via the plurality of apertures or openings 39), through the second chamber 36, and out the blood outlet 14.

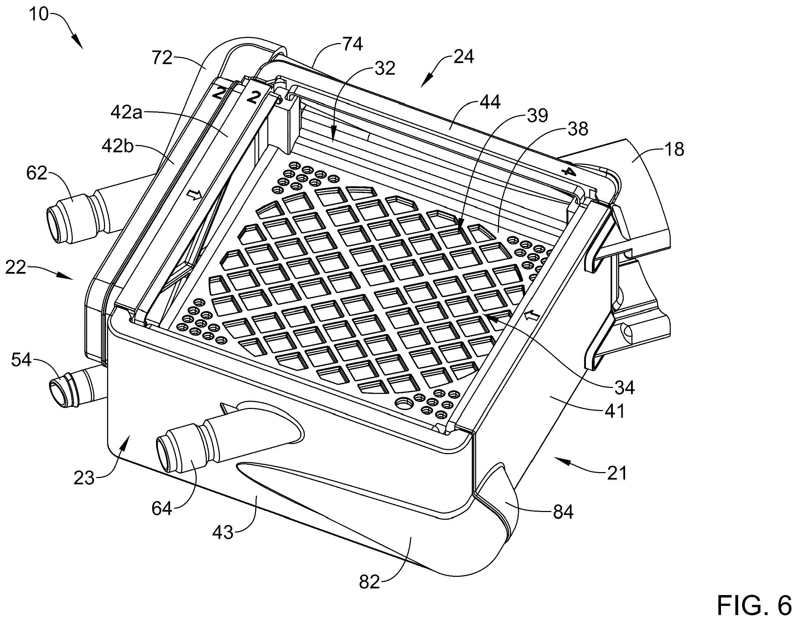

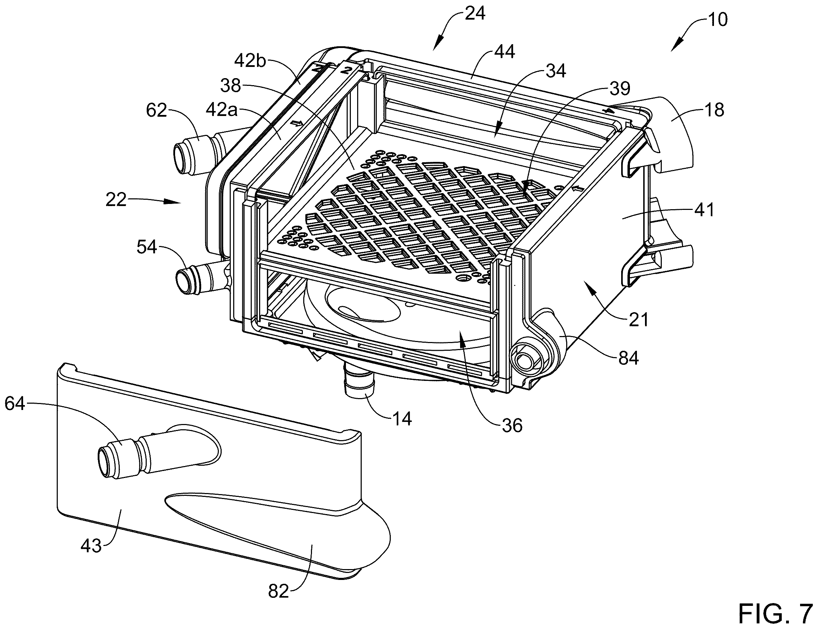

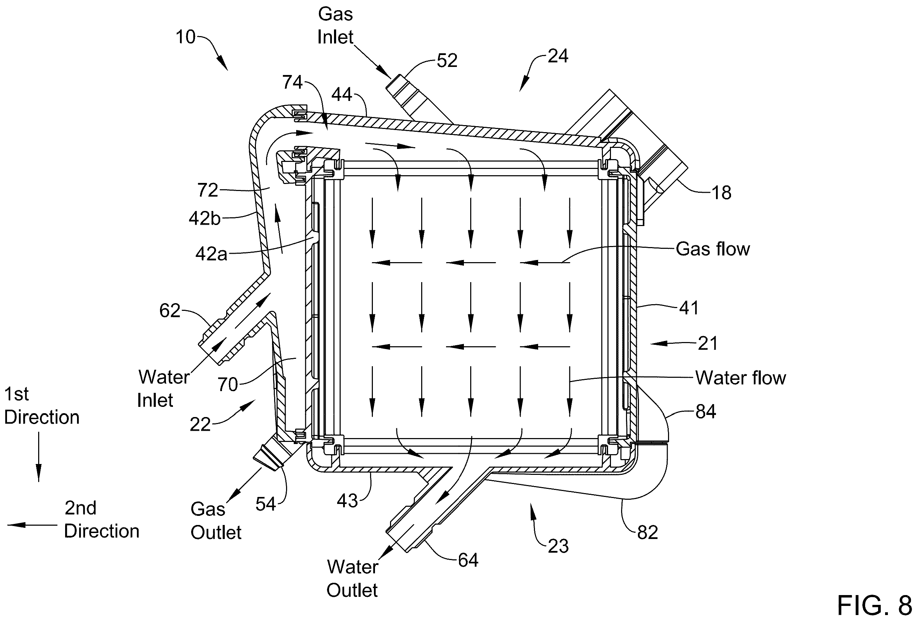

Further discussion of the first chamber 34 will be described in association with FIGS. 6-8. FIG. 6 is a perspective top view of the housing 10 of the oxygenator 2 with the top panel 40 removed to view the upper, first chamber 34 of the interior 32 of the oxygenator 2. FIG. 7 is a perspective top view of the housing 10 of the oxygenator 2 with the top panel 40 and the third side panel 43 removed to view the first chamber 34 and the lower, second chamber 36 of the interior 32 of the oxygenator 2. FIG. 8 is a cross-sectional view through the first chamber 34, described hereinafter as the gas-and-heat exchange chamber, of the oxygenator 2 illustrating a gas exchange fluid flow path and a heat exchange fluid flow path therethrough.

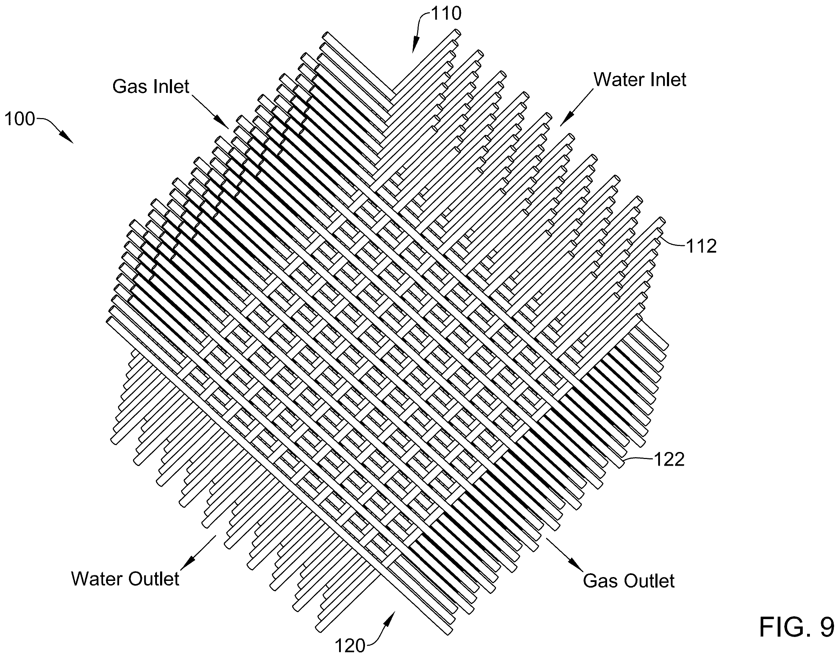

The gas-and-heat exchange chamber 34 may be configured to provide both gas exchange between a gas (e.g., oxygen) and the blood flowing through the gas-and-heat exchange chamber 34, and also be configured to provide heat exchange between a heat exchange fluid (e.g., water) and the blood flowing through the gas-and-heat exchange chamber 34. For instance, the gas-and-heat exchange chamber 34 may include a plurality of heat exchange fibers and a plurality gas exchange fibers arranged as a gas-and-heat exchange section of fibers in the gas-and-heat exchange chamber 34. Blood may flow around the plurality of heat exchange fibers and the plurality gas exchange fibers as the blood flows through the gas-and-heat exchange chamber 34. One possible arrangement of the gas-and-heat exchange section of fibers is illustrated in FIG. 9.

As shown in FIG. 9, the gas-and-heat exchange fiber section 100 may include a plurality of heat exchange fibers 112 and a plurality of gas exchange fibers 122. In some instances, the plurality of heat exchange fibers 112 may be arranged in one or more, or a plurality of heat exchange fiber layers 110 and/or the plurality of gas exchange fibers 122 may be arranged in one or more, or a plurality of gas exchange fiber layers 120. The heat exchange fiber layers 110 may be alternated with the gas exchange fiber layers 120 along a thickness dimension of the gas-and-heat exchange fiber section 100, for example. For instance, the plurality of heat exchange fibers 112 in each of the heat exchange fiber layers 110 in the gas-and-heat exchange chamber 34 may be arranged in a first direction, and the plurality of gas exchange fibers 122 in each of the gas exchange fiber layers 120 in the gas-and-heat exchange chamber 34 may be arranged in a second direction. The first direction may be generally orthogonal or perpendicular to the second direction, in some instances. Accordingly, the plurality of heat exchange fibers 112 may be arranged substantially perpendicular to the plurality of gas exchange fibers 122 in the gas-and-heat exchange chamber 34. In some instances, the first direction may be at an angle of between about 60 degrees to 90 degrees or between about 75 degrees to 90 degrees, to the second direction.

The heat exchange fibers 112 may extend in the first direction between the fourth side 24 and the third side 23 of the housing 10 in the gas-and-heat exchange chamber 34. For instance, inflow ends of the heat exchange fibers 112 may be encased in potting proximate the fourth side panel 44 while outflow ends of the heat exchange fibers 112 may be encased in potting proximate the third side panel 43. In the case of reverse flow, the ends of the heat exchange fibers 112 at the third side panel 43 may be considered the inflow ends of the heat exchange fibers 112, and the ends of the heat exchange fibers 112 at the fourth side panel 44 may be considered the outflow ends of the heat exchange fibers 112.

The gas exchange fibers 122 may extend in the second direction between the first side 21 and the second side 22 of the housing 10 in the gas-and-heat exchange chamber 34. For instance, inflow ends of the gas exchange fibers 122 may be encased in potting proximate the first side panel 41 while outflow ends of the gas exchange fibers 122 may be encased in potting proximate the second side panel (e.g., the second side inner panel 42a).

Returning to FIG. 8, the flow pathway of the heat exchange fluid (e.g., water) will be further described. The heat exchange fluid may enter the oxygenator 2 through the first heat exchange fluid port 62 (i.e., the heat exchange fluid inlet) arranged on the second side 22 of the housing 10. The heat exchange fluid may then enter an insulating barrier chamber 70 extending along the second side 22 of the housing 10. The heat exchange fluid may flow through the insulating barrier chamber 70 into a heat exchange fluid passage 72 defined along the second side 22, and thereafter, into a heat exchange fluid passage 74 defined along the fourth side 24. The heat exchange fluid passage 74 may extend a substantial length of the fourth side 24, permitting the heat exchange fluid to reach the inflow ends of the plurality of heat exchange fluid fibers arranged in the gas-and-heat exchange chamber 34. The heat exchange fluid may then pass through the heat exchange fibers 112 from the fourth side 24 to the third side 23 in the first direction, as shown in FIG. 8. The heat exchange fluid may then flow out of the outflow ends of the heat exchange fibers at the third side 23 and exit the oxygenator 2 (e.g., may exit the gas-and-heat exchange chamber 34 of the housing 10 of the oxygenator 2) through the second heat exchange fluid port 64 (i.e., the heat exchange fluid outlet) extending from the third side 23.

In an alternative arrangement in which the direction of heat exchange fluid is reversed, the heat exchange fluid may enter the oxygenator 2 through the second heat exchange fluid port 64 (i.e., the heat exchange fluid inlet) and flow into the inflow ends of the heat exchange fibers at the third side 23. The heat exchange fluid may then pass through the heat exchange fibers 112 from the third side 23 to the fourth side 24 in a direction opposite the first direction shown in FIG. 8, and flow out of the outflow ends of the heat exchange fibers at the fourth side 24. The heat exchange fluid may then flow through the heat exchange fluid passage 74 defined along the fourth side 24 and into the insulating barrier chamber 70 via the heat exchange fluid passage 72 defined along the second side 22. The heat exchange fluid may then exit the oxygenator 2 through the first heat exchange fluid port 62 (i.e., the heat exchange fluid outlet) extending from the second side 22.

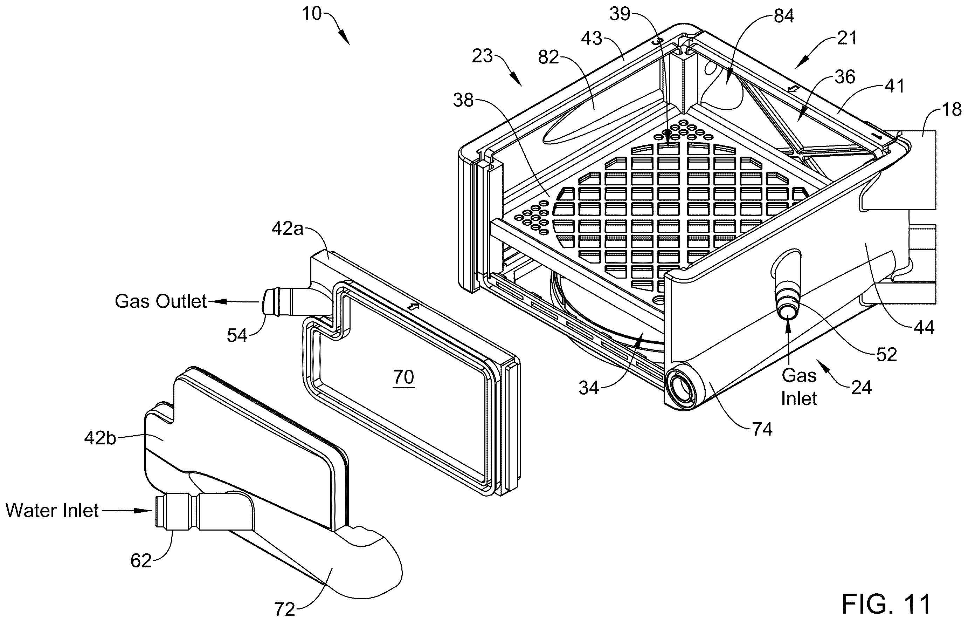

The insulating barrier chamber 70 may be a space defined between the second side inner panel 42a and the second side outer panel 42b. For example, an outward facing surface of the second side inner panel 42a may be spaced away from an inward facing surface of the second side outer panel 42b, defining the insulating barrier chamber 70 therebetween. The insulating barrier chamber 70 may extend across a majority of the second side 22 of the housing 10, and in some instances, the insulating barrier chamber 70 may extend across substantially the entire second side 22 of the housing 10. As shown in FIG. 11, the insulating barrier chamber 70 may extend substantially the entire height of the second side 22, between the top side and the bottom side of the housing 10, such that the insulating barrier chamber 70 is arranged exterior of the first chamber 34 and the second chamber 36. The insulating barrier chamber 70 may be in fluid communication with the heat exchange fluid passage 72, such that fluid exiting the insulating barrier chamber 70 enters (e.g., directly enters) into the heat exchange fluid passage 72, or in the case of reverse flow, fluid exiting the heat exchange fluid passage 72 enters (e.g., directly enters) into the insulating barrier chamber 70. In some instances the heat exchange fluid passage 72 may be integrally formed in the second side outer panel 42b, or the heat exchange fluid passage 72 may be formed separately. The heat exchange fluid passage 72 may be fluidly coupled (e.g., in fluid communication with) to the heat exchange fluid passage 74. In some instances the heat exchange fluid passage 74 may be integrally formed in the fourth side panel 44, or the heat exchange fluid passage 74 may be formed separately. The heat exchange fluid passage 74 may extend across a majority of the fourth side 24 of the housing 10 (e.g., may extend across a majority of the length of the fourth side panel 44), and in some instances, the heat exchange fluid passage 74 may extend across substantially the entire fourth side 24 of the housing 10 (e.g., may extend across substantially the entire length of the fourth side panel 44). Accordingly, the heat exchange fluid passage 72 and the heat exchange fluid passage 74 may route the heat exchange fluid around the perimeter of the housing 10 from the second side 22 to the adjacent fourth side 24 of the housing 10, or in the case of reverse flow, from the fourth side 24 to the second side 22. For instance, the heat exchange fluid passage 72 and the heat exchange fluid passage 74 may route the heat exchange fluid from the first heat exchange fluid port 62 (extending from the second side 22), around a perimeter of the housing 10 to the inflow ends of the heat exchange fibers 112 arranged along the fourth side 24 of the housing 10 in the gas-and-heat exchange chamber 34. In the case of reverse flow, the heat exchange fluid passage 72 and the heat exchange fluid passage 74 may route the heat exchange fluid from the outflow ends of the heat exchange fibers 112 arranged along the fourth side 24 of the housing 10 in the gas-and-heat exchange chamber 34, around a perimeter of the housing 10 to the first heat exchange fluid port 62 (extending from the second side 22).

The flow pathway of the gas exchange fluid (e.g., oxygen or oxygenating gas) through the gas exchange fibers 122 in the gas-and-heat exchange chamber 34 is also illustrated in FIG. 8. The gas exchange fluid may flow from the first side 21 to the second side 22 in the second direction, as shown in FIG. 8. The second direction may be substantially orthogonal or perpendicular to the first direction. Accordingly, the flow pathway of the gas exchange fluid through the gas-and-heat exchange chamber 34 may be substantially orthogonal or perpendicular to the flow pathway of the heat exchange fluid through the gas-and-heat exchange chamber 34. The gas exchange fluid may then flow out of the outflow ends of the gas exchange fibers 122 at the second side 22 and exit the oxygenator 2 (e.g., may exit the gas-and-heat exchange chamber 34 of the housing 10 of the oxygenator 2) through the gas exchange fluid outlet 54 extending from the second side 22. The flow pathway of the gas exchange fluid prior to reaching the gas-and-heat exchange chamber 34 (e.g., prior to entering the inflow ends of the gas exchange fibers 122 in the gas-and-heat exchange chamber 34) will be described further herein.

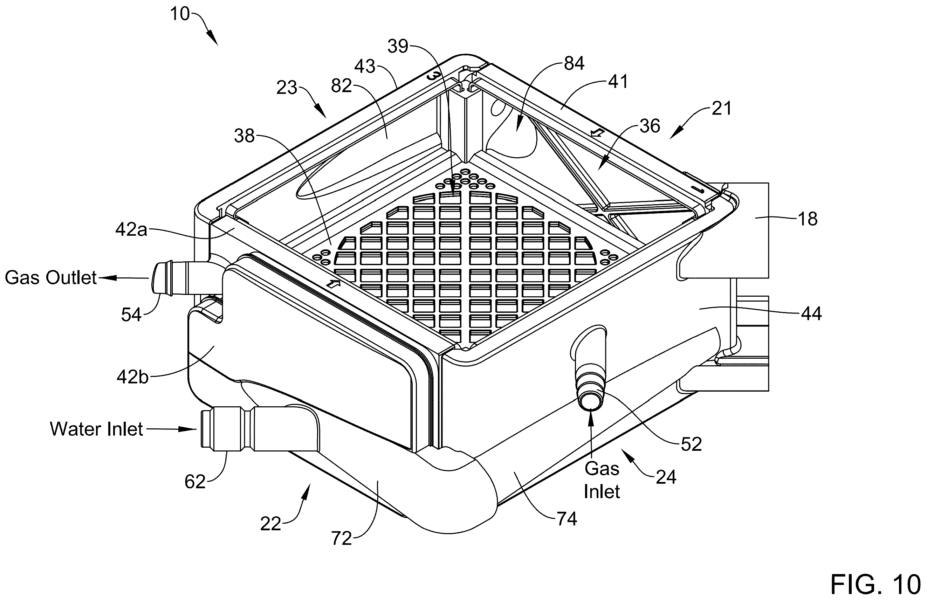

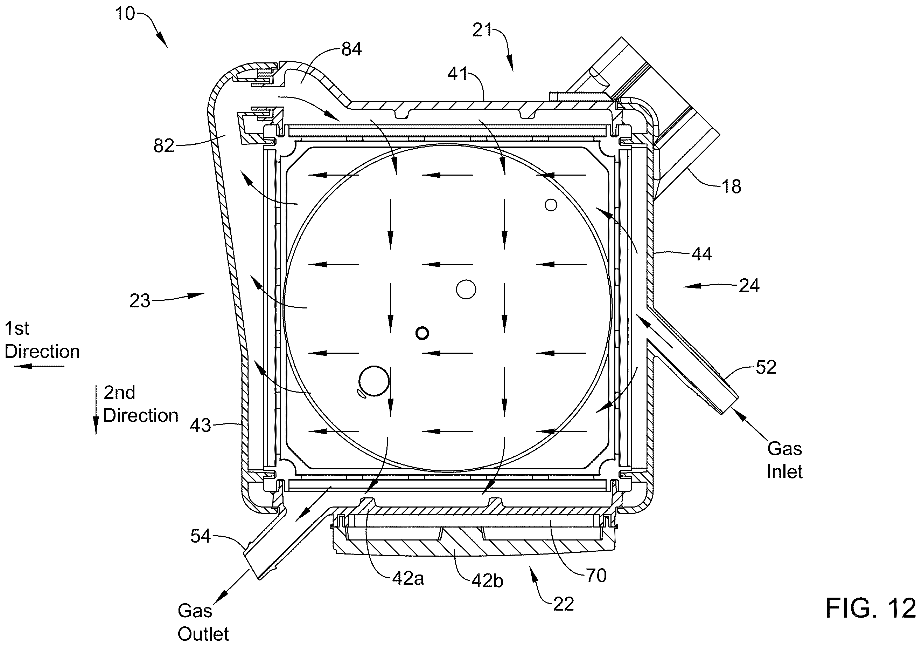

Further discussion of the second chamber 36 will be described in association with FIGS. 10-12. FIG. 10 is a perspective bottom view of the housing 10 of the oxygenator 2 with the bottom panel 50 removed to view the second chamber 36 of the interior 32 of the oxygenator 2. FIG. 11 is a perspective bottom view of the housing 10 of the oxygenator 2 with the bottom panel 50 and the second side panel (e.g., the second side inner panel 42a and the second side outer panel 42b) removed to view the first chamber 34 and the second chamber 36 of the interior 32 of the oxygenator 2. FIG. 12 is a cross-sectional view through the second chamber 36, described hereinafter as the gas exchange chamber, of the oxygenator 2 illustrating a gas exchange fluid flow path therethrough.

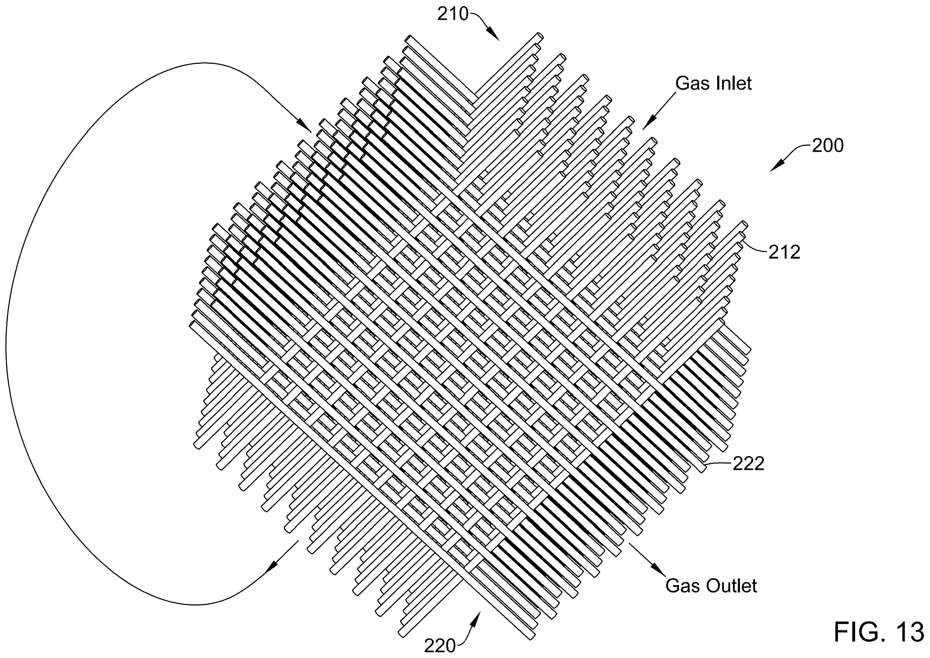

The gas exchange chamber 36 may be configured to provide additional gas exchange between a gas exchange fluid (e.g., oxygen) and the blood flowing through the gas exchange chamber 36. For instance, the gas exchange chamber 36 may include a plurality gas exchange fibers arranged as a gas exchange section of fibers in the gas exchange chamber 36. Blood may flow around the plurality of gas exchange fibers as the blood flows through the gas exchange chamber 36. One possible arrangement of the gas exchange section of fibers is illustrated in FIG. 13.

As shown in FIG. 13, the gas exchange fiber section 200 may include a first plurality of gas exchange fibers 212 and a second plurality of gas exchange fibers 222. In some instances, the first plurality of gas exchange fibers 212 may be arranged in one or more, or a plurality of first gas exchange fiber layers 210 and/or the second plurality of gas exchange fibers 222 may be arranged in one or more, or a plurality of second gas exchange fiber layers 220. The first gas exchange fiber layers 210 may be alternated with the second gas exchange fiber layers 220 along a thickness dimension of the gas exchange fiber section 200, for example. For instance, the first plurality of gas exchange fibers 212 in each of the first gas exchange fiber layers 210 in the gas exchange chamber 36 may be arranged in a first direction, and the second plurality of gas exchange fibers 222 in each of the second gas exchange fiber layers 220 in the gas exchange chamber 36 may be arranged in a second direction. The first direction may be generally orthogonal or perpendicular to the second direction, in some instances. Accordingly, the first plurality of gas exchange fibers 212 may be arranged substantially perpendicular to the second plurality of gas exchange fibers 222 in the gas exchange chamber 36. In some instances, the first direction may be at an angle of between about 60 degrees to 90 degrees or between about 75 degrees to 90 degrees, to the second direction.

The first plurality of gas exchange fibers 212 may extend in the first direction between the fourth side 24 and the third side 23 of the housing 10 in the gas exchange chamber 36. For instance, inflow ends of the first plurality of gas exchange fibers 212 may be encased in potting proximate the fourth side panel 44 while outflow ends of the first plurality of gas exchange fibers 212 may be encased in potting proximate the third side panel 43.

The second plurality of gas exchange fibers 222 may extend in the second direction between the first side 21 and the second side 22 of the housing 10 in the gas exchange chamber 36. For instance, inflow ends of the second plurality of gas exchange fibers 222 may be encased in potting proximate the first side panel 41 while outflow ends of the second plurality of gas exchange fibers 222 may be encased in potting proximate the second side panel (e.g., the second side inner panel 42a).

The direction or orientation of the second plurality of gas exchange fibers 222 in the gas exchange chamber 36 may be the same as the gas exchange fibers 122 in the gas-and-heat exchange chamber 34. In other words, the second plurality of gas exchange fibers 222 in the gas exchange chamber 36 may be arranged parallel to, or at least substantially parallel to, the gas exchange fibers 122 in the gas-and-heat exchange chamber 34, with the inflow ends of both the second plurality of gas exchange fibers 222 in the gas exchange chamber 36 and the gas exchange fibers 122 in the gas-and-heat exchange chamber 34 encased in potting proximate the first side 21, and with the outflow ends of both the second plurality of gas exchange fibers 222 in the gas exchange chamber 36 and the gas exchange fibers 122 in the gas-and-heat exchange chamber 34 encased in potting proximate the second side 22.

Returning to FIG. 12, the flow pathway of the gas exchange fluid (e.g., oxygen or oxygenating gas) will be further described. The gas exchange fluid may enter the oxygenator 2 through the gas exchange fluid inlet 52 arranged on the fourth side 24 of the housing 10. The gas exchange fluid may then flow into the inflow ends of the first plurality of gas exchange fibers 212 arranged in the gas exchange chamber 36. The gas exchange fluid may then pass through the first plurality of gas exchange fibers 212 from the fourth side 24 to the third side 23 in the first direction, as shown in FIG. 12. The gas exchange fluid may then flow out of the outflow ends of the first plurality of gas exchange fibers 212 at the third side 23 into a gas exchange fluid passage 82 extending along the third side 23.

The gas exchange fluid passage 82 may be fluidly coupled (e.g., in fluid communication with) to a gas exchange fluid passage 84 extending along the first side 21. In some instances, the gas exchange fluid passage 82 may be integrally formed in the third side panel 43, or the gas exchange fluid passage 82 may be formed separately. In some instances, the gas exchange fluid passage 84 may be integrally formed in the first side panel 41, or the gas exchange fluid passage 84 may be formed separately. Accordingly, the gas exchange fluid passage 82 and the gas exchange fluid passage 84 may route the gas exchange fluid around the perimeter of the housing 10 from the third side 23 to the adjacent first side 21 of the housing 10. For instance, the gas exchange fluid passage 82 and the gas exchange fluid passage 84 may route the gas exchange fluid from the outflow ends of the first plurality of gas exchange fibers 212 in the gas exchange chamber 36, around a perimeter of the housing 10 to the inflow ends of the second plurality of gas exchange fibers 222 in the gas exchange chamber 36.

The gas exchange fluid (e.g., oxygen or oxygenating gas) may then flow from the first side 21 to the second side 22 in the second direction, as shown in FIG. 12. The second direction may be substantially orthogonal or perpendicular to the first direction. Accordingly, the flow pathway of the gas exchange fluid through the first plurality of gas exchange fibers 212 in the gas exchange chamber 36 may be substantially orthogonal or perpendicular to the flow pathway of the gas exchange fluid through the second plurality of gas exchange fibers 222 in the gas exchange chamber 36. Accordingly, the gas exchange fluid may sequentially pass through the first plurality of gas exchange fibers 212 in the first direction, and then pass through the second plurality of gas exchange fibers 222 in the second direction, improving the efficiency of the O₂ and CO₂ gas exchange between the gas exchange fluid and the blood. The gas exchange fluid may then flow out of the outflow ends of the second plurality of gas exchange fibers 222 at the second side 22 and exit the oxygenator 2 (e.g., may exit the gas exchange chamber 36 of the housing 10 of the oxygenator 2) through the gas exchange fluid outlet 54 extending from the second side 22.

Furthermore, the gas exchange fluid passage 82 and the gas exchange fluid passage 84 may route the gas exchange fluid from the outflow ends of the first plurality of gas exchange fibers 212 in the gas exchange chamber 36, around a perimeter of the housing 10 to the inflow ends of the gas exchange fibers 122 in the gas-and-heat exchange chamber 34. Accordingly, the gas exchange fluid passage 82 and the gas exchange fluid passage 84 may be in fluid communication with both the inflow ends of the gas exchange fibers 122 in the gas-and-heat exchange chamber 34 and the second plurality of gas exchange fibers 222 in the gas exchange chamber 36. In other words, the gas exchange fluid exiting the gas exchange fluid passage 84 at the first side 21 may be dispersed or divided between the inflow ends of the gas exchange fibers 122 in the gas-and-heat exchange chamber 34 and the inflow ends of the second plurality of gas exchange fibers 222 in the gas exchange chamber 36. Thus, the gas exchange fluid may pass through the first plurality of gas exchange fibers 212 in the gas exchange chamber 36 in the first direction prior to passing through the gas exchange fibers 122 in the gas-and-heat exchange chamber 34 in the second direction.

The sequential flow path of the gas exchange fluid through the oxygenator 2 may improve the gas exchange between the gas exchange fluid and the blood, as well as provide other beneficial effects. The gas exchange fluid may pass through the first plurality of gas exchange fibers 212 in the gas exchange chamber 36 from the fourth side 24 to the third side 23 in the first direction, as shown in FIG. 12, at a first velocity, but then the quantity of gas exchange fluid passing through the first plurality of gas exchange fibers 212 is divided amongst the second plurality of gas exchange fibers 222 in the gas exchange chamber 36 and the plurality of gas exchange fibers 122 in the gas-and-heat exchange chamber 34 as the gas exchange fluid passes from the first side 21 to the second side 22 in the second direction, as shown in FIGS. 8 and 12. As the sum of the quantity of second plurality of gas exchange fibers 222 in the gas exchange chamber 36 and the plurality of gas exchange fibers 122 in the gas-and-heat exchange chamber 34 (or at least the sum of the cross-sectional area of the lumens thereof) is greater than the quantity of the first plurality of gas exchange fibers 212 in the gas exchange chamber 36 (or at least the total cross-sectional area of the lumens thereof), the velocity of the gas exchange fluid flowing in the second direction is reduced. Thus, the sequential flow path of the gas exchange fluid through the oxygenator 2 is such that the velocity of the gas exchange fluid flowing in the first direction across the housing 10 from the fourth side 24 to the third side 23 is greater than the velocity of the gas exchange fluid flowing in the second direction across the housing 10 from the first side 21 to the second side 22.

In some instances, the velocity of the gas exchange fluid flowing in the first direction across the housing 10 from the fourth side 24 to the third side 23 is 20% or more greater than the velocity of the gas exchange fluid flowing in the second direction across the housing 10 from the first side 21 to the second side 22. In some instances, the velocity of the gas exchange fluid flowing in the first direction across the housing 10 from the fourth side 24 to the third side 23 is 25% or more greater than the velocity of the gas exchange fluid flowing in the second direction across the housing 10 from the first side 21 to the second side 22. In some instances, the velocity of the gas exchange fluid flowing in the first direction across the housing 10 from the fourth side 24 to the third side 23 is 30% or more greater than the velocity of the gas exchange fluid flowing in the second direction across the housing 10 from the first side 21 to the second side 22. In some instances, the velocity of the gas exchange fluid flowing in the first direction across the housing 10 from the fourth side 24 to the third side 23 is 50% or more greater than the velocity of the gas exchange fluid flowing in the second direction across the housing 10 from the first side 21 to the second side 22.

In some instances, the first velocity of the gas exchange fluid in the first direction may be about 1.2 times or more the second velocity of the gas exchange fluid in the second direction. In some instances, the first velocity of the gas exchange fluid in the first direction may be about 1.25 times or more the second velocity of the gas exchange fluid in the second direction. In some instances, the first velocity of the gas exchange fluid in the first direction may be about 1.3 times or more the second velocity of the gas exchange fluid in the second direction. In some instances, the first velocity of the gas exchange fluid in the first direction may be about 1.5 times or more the second velocity of the gas exchange fluid in the second direction. In some instances, the first velocity of the gas exchange fluid in the first direction may be about 2 times or more the second velocity of the gas exchange fluid in the second direction. Depending on the quantity, size, and arrangement of gas exchange fibers, ratio of the first velocity of the gas exchange fluid in the first direction to the second velocity of the gas exchange fluid in the second direction may be 1.2:1 or more, 1.25:1 or more, 1.3:1 or more, 1.4: 1 or more, 1.5:1 or more, 1.75:1, or more, 2:1 or more, 2.25:1, or more, or 2.5:1 or more, for example.

Said differently, since the quantity of gas exchange fluid passing through the first plurality of gas exchange fibers 212 is then divided amongst the second plurality of gas exchange fibers 222 in the gas exchange chamber 36 and the plurality of gas exchange fibers 122 in the gas-and-heat exchange chamber 34, the flow rate of the gas exchange fluid through the first plurality of gas exchange fibers 212 in the gas exchange chamber 36 from the fourth side 24 to the third side 23 in the first direction, as shown in FIG. 12, may be greater than the flow rate of the gas exchange fluid through either of the second plurality of gas exchange fibers 222 in the gas exchange chamber 36 and the plurality of gas exchange fibers 122 in the gas-and-heat exchange chamber 34 as the gas exchange fluid passes from the first side 21 to the second side 22 in the second direction, as shown in FIGS. 8 and 12.

The insulating barrier chamber 70 provided with the housing 10 of the oxygenator 2 may provide additional benefits. For example, the presence of the insulating barrier chamber 70 arranged between gas exchange fluid outlet 54 and the outflow ends of the plurality of gas exchange fibers 122 in the gas-and-heat exchange chamber 34 and the second plurality of gas exchange fibers 222 in the gas exchange chamber 36 may reduce or eliminate vapor condensation that may otherwise form in the oxygenator 2 proximate the gas exchange fluid outlet 54. Thus, the heat exchange fluid flowing through the oxygenator 2 may act as both an inflating barrier to reduce or eliminate vapor condensation proximate the gas exchange fluid outlet 54, and may provide heating and/or cooling of the blood flowing through the oxygenator 2.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The scope of the disclosure is, of course, defined in the language in which the appended claims are expressed.

## Claims

1. An oxygenator, comprising:
a housing defining an internal chamber;
a plurality gas exchange fibers within in the internal chamber; the plurality of gas exchange fibers including one or more layers of gas exchange fibers extending in a first direction and one or more layers of gas exchange fibers extending in a second direction, the second direction being generally orthogonal to the first direction;
a gas exchange fluid inlet;
a gas exchange fluid outlet; and
a gas exchange fluid pathway for passing a gas through the plurality of gas exchange fibers to oxygenate blood within the internal chamber, the gas exchange fluid pathway passing sequentially from the gas exchange fluid inlet, through the one or more layers of gas exchange fibers extending in the first direction, through the one or more layers of gas exchange fibers extending in the second direction, and out the gas exchange fluid outlet.

2. The oxygenator of claim 1, wherein the housing includes a first side, a second side opposite the first side, a third side extending between the first side and the second side, and a fourth side extending between the first side and the second side, and opposite the third side; and
wherein the gas exchange fluid pathway passes through the one or more layers of gas exchange fibers extending in the first direction from the fourth side to the third side, and then the gas exchange fluid pathway passes through the one or more layers of gas exchange fibers extending in the second direction from the first side to the second side.

3. The oxygenator of claim 2, wherein a first velocity of a gas exchange fluid flowing through the one or more layers of gas exchange fibers extending in the first direction from the fourth side to the third side is greater than a second velocity of the gas exchange fluid flowing through the one or more layers of gas exchange fibers extending in the second direction from the first side to the second side.

4. The oxygenator of claim 3, wherein the first velocity is 1.25 times or more the velocity of the second velocity.

5. The oxygenator of claim 1, further comprising a gas exchange fluid passage defined in the housing between gas outflow ends of the one or more layers of gas exchange fibers extending in the first direction and gas inflow ends of the one or more layers of gas exchange fibers extending in the second direction.

6. The oxygenator of claim 1, wherein the internal chamber is a gas exchange chamber, and the housing includes a second internal chamber which is a gas-and-heat exchange chamber, wherein the gas-and-heat exchange chamber includes a plurality of gas exchange fibers within the gas-and-heat exchange chamber and a plurality of heat exchange fibers within the gas-and-heat exchange chamber.

7. The oxygenator of claim 6, further comprising a partition between the gas exchange chamber and the gas-and-heat exchange chamber.

8. The oxygenator of claim 6, wherein the housing includes a blood inlet in fluid communication with the gas-and-heat exchange chamber and a blood outlet in fluid communication with the gas exchange chamber.

9. The oxygenator of claim 8, wherein a blood flow path passes sequentially from the blood inlet, through the gas-and-heat exchange chamber, through the gas exchange chamber, and out the blood outlet.

10. The oxygenator of claim 6, wherein the plurality of heat exchange fibers in the gas-and-heat exchange chamber are arranged in the first direction, and the plurality of gas exchange fibers in the gas-and-heat exchange chamber are arranged in the second direction.

11. The oxygenator of claim 1, wherein the housing includes a first side, a second side opposite the first side, a third side extending between the first side and the second side, and a fourth side extending between the first side and the second side, and opposite the third side;
wherein the gas exchange fluid pathway passes through the one or more layers of gas exchange fibers extending in the first direction from the fourth side to the third side, and then the gas exchange fluid pathway passes through the one or more layers of gas exchange fibers extending in the second direction from the first side to the second side; and
wherein the second side includes an insulating fluid barrier chamber configured to be filled with a flowing heat exchange fluid.

12. The oxygenator of claim 11, wherein the housing includes a heat exchange fluid port in fluid communication with the insulating fluid barrier chamber.

13. The oxygenator of claim 12, wherein the heat exchange fluid port and the gas exchange fluid outlet are both located on the second side of the housing.

14. The oxygenator of claim 11, wherein the insulating fluid barrier chamber is positioned between an outflow of the one or more layers of gas exchange fibers extending in the second direction at the second side and an exterior of the second side of the housing.

15. An oxygenator, comprising:
a housing defining an interior, the interior including a gas exchange chamber and a gas-and-heat exchange chamber,
a partition between the gas exchange chamber and the gas-and-heat exchange chamber;
a plurality of gas exchange fibers within the gas exchange chamber;
a plurality of gas exchange fibers within the gas-and-heat exchange chamber; and
a plurality of heat exchange fibers arranged in the gas-and-heat exchange chamber.

16. The oxygenator of claim 15, wherein the plurality of gas exchange fibers within the gas exchange chamber include:
one or more layers of gas exchange fibers extending in a first direction; and
one or more layers of gas exchange fibers extending in a second direction,
wherein the second direction is generally orthogonal to the first direction.

17. The oxygenator of claim 16, wherein a gas exchange fluid pathway for passing a gas through the plurality of gas exchange fibers in the gas exchange chamber to oxygenate blood within the in the gas exchange chamber sequentially passes through the one or more layers of gas exchange fibers extending in the first direction and thereafter through the one or more layers of gas exchange fibers extending in the second direction.

18. The oxygenator of claim 17, wherein a first velocity of a gas exchange fluid flowing through the one or more layers of gas exchange fibers extending in the first direction is greater than a second velocity of the gas exchange fluid flowing through the one or more layers of gas exchange fibers extending in the second direction.

19. The oxygenator of claim 17, further comprising a gas exchange fluid passage defined in the housing between gas outflow ends of the one or more layers of gas exchange fibers extending in the first direction and gas inflow ends of the one or more layers of gas exchange fibers extending in the second direction.

20. The oxygenator of claim 16, wherein:
the plurality of heat exchange fibers arranged in the gas-and-heat exchange chamber include one or more layers of heat exchange fibers extending in the first direction; and
the plurality of gas exchange fibers arranged in the gas-and-heat exchange chamber include one or more layers of gas exchange fibers extending in the second direction.

21. An oxygenator, comprising:
a housing including a first side, a second side opposite the first side, a third side extending between the first side and the second side, and a fourth side extending between the first side and the second side, and opposite the third side, the housing defining an interior;
a plurality of gas exchange fibers within the interior of the housing, the plurality of gas exchange fibers extending between the first side and the second side with gas outflow from the plurality of gas exchange fibers along the second side;
a plurality of heat exchange fibers within the interior of the housing;
a gas exchange fluid inlet in fluid communication with the plurality of gas exchange fibers;
a gas exchange fluid outlet in fluid communication with the plurality of gas exchange fibers;
a heat exchange fluid inlet in fluid communication with the heat exchange fibers; and
a heat exchange fluid outlet in fluid communication with the heat exchange fibers;
wherein the second side includes an insulating fluid barrier chamber configured to be filled with a flowing heat exchange fluid from the heat exchange fluid inlet.

22. The oxygenator of claim 21, wherein the insulating fluid barrier is fluidly arranged between the heat exchange fluid inlet and inflow ends of the plurality of heat exchange fibers.

23. The oxygenator of claim 21, wherein the inflow ends of the plurality of heat exchange fibers are located along the fourth side, and wherein the plurality of heat exchange fibers extend between the fourth side and the third side.

24. An oxygenator, comprising:
a housing defining an interior;
a plurality gas exchange fibers within in the interior; the plurality of gas exchange fibers including one or more layers of gas exchange fibers extending in a first direction and one or more layers of gas exchange fibers extending in a second direction, the second direction being generally orthogonal to the first direction;
a gas exchange fluid inlet;
a gas exchange fluid outlet; and
a gas exchange fluid pathway for passing a gas from the gas exchange fluid inlet, through the plurality of gas exchange fibers to oxygenate blood within the interior of the housing, to the gas exchange fluid outlet;
wherein a first velocity of a gas exchange fluid flowing through the one or more layers of gas exchange fibers extending in the first direction is greater than a second velocity of the gas exchange fluid flowing through the one or more layers of gas exchange fibers extending in the second direction.

25. The oxygenator of claim 24, wherein the first velocity is 1.25 times or more the velocity of the second velocity.
